# EUROPÄISCHE PATENTSCHRIFT

(11) **EP 3 527 232 B1**
(45) Veröffentlichungstag und Bekanntmachung des Hinweises auf die Patenterteilung: **15.09.2021**
(21) Anmeldenummer: 18157512.7
(22) Anmeldetag: 19.02.2018
(51) Int. Cl.: A61L 2/26

(54) **ROLLCONTAINERSYSTEM UND VERFAHREN ZUR VERSORGUNG EINER REINRAUMSTATION MIT STERILEN GEGENSTÄNDEN**
ROLL CONTAINER SYSTEM AND METHOD FOR SUPPLYING A CLEAN AIR STATION WITH STERILE OBJECTS
SYSTÈME DE CONTENEUR ROULANT ET PROCÉDÉ D'APPROVISIONNEMENT D'UNE STATION DE SALLE PROPRE EN OBJETS STÉRILES

(43) Veröffentlichungstag der Anmeldung: 21.08.2019
(73) Patentinhaber: Cosanum AG, 8952 Schlieren (CH)
(72) Erfinder: SCHEFER, Thomas, 8952 Schlieren (CH)
(74) Vertreter: Schmauder & Partner AG Patent- & Markenanwälte VSP

(56) Entgegenhaltungen:
- EP-A1- 2 090 324
- CN-A- 107 583 069
- JP-A- 2016 123 775

## Beschreibung

### Technisches Gebiet

Die vorliegende Erfindung betrifft ein Rollcontainersystem, insbesondere zur Versorgung einer Reinraumstation, sowie ein Verfahren zur Versorgung einer Reinraumstation mit sterilen Gegenständen.

### Stand der Technik

In sehr zahlreichen Anwendungsbereichen werden sterile Gegenstände aller Art eingesetzt. Neben einer Sterilisation direkt am Einsatzort ist es zweckmässig oder notwendig, die Gegenstände in sterilem Zustand auswärts, d.h. bei einem Hersteller oder Verarbeiter bereitzustellen und an den Einsatzort zu transportieren. Es versteht sich, dass bei diesem Vorgang kein Verlust der Sterilität eintreten darf.

Ein Anwendungsbereich mit besonders umfangreichem Bedarf an zugelieferten sterilen Gegenständen ist der medizinische Bereich, wozu insbesondere die Belieferung von Krankenhäusern mit sterilen Gegenständen für chirurgische Eingriffe gehört. Zu diesen Gegenständen gehören namentlich auch sterilisierte Abdecktücher aller Art, die in sehr grosser Menge benötigt werden.

Für das Transportieren von sterilen oder sonstwie reinen Gegenständen von einer ersten reinen Umgebung zu einer zweiten reinen Umgebung durch eine unreine Zwischenumgebung sind bereits verschiedene technische Lösungen vorgeschlagen und auch eingesetzt worden. Insbesondere beschreibt die DE 19840561 A1 eine entsprechende Vorrichtung zum Transportieren von frisch sterilisierten bzw. autoklavierten Versuchstierkäfigen von einem Autoklaven zu einer Arbeitsstation. Die dort beschriebene Vorrichtung umfasst einen als Rollcontainer ausgebildeten Transportbehälter mit einer abdichtenden Verschlusseinrichtung, die als einzelner Verschluss ausgebildet ist. Die beschriebene Lösung beruht auf einem Konzept, wonach der Rollcontainer am Zielort als Ganzes in eine Autoklavenvorrichtung eingeführt wird. Dies ist für gewisse Arten von Gegenständen wie beispielsweise Versuchstierkäfige geeignet. Das Autoklavieren eines ganzen Rollcontainers einschliesslich darin befindlicher Gegenstände ist allerdings nur für begrenzte Anwendungsbereiche geeignet.

Ein gattungsgemässes Transfercontainersystem ist in der EP 2 090 324 A1 beschrieben. Dieses umfasst einen dichtend verschliessbaren äusseren Rollcontainer und mindestens einen darin ein- und ausfahrbaren inneren Rollcontainer, wobei der innere Rollcontainer ein im wesentlichen quaderförmiges Innengehäuse sowie mindestens eine Ablagefläche aufweist, und wobei der äussere Rollcontainer ein im wesentlichen quaderförmiges Aussengehäuse, welches auf einem zweiten Lenkrollenpaar sowie einem in einer Laufrichtung (La) des äusseren Rollcontainers ausgerichteten zweiten Zusatzrollenpaar läuft, und eine quer zur Laufrichtung des äusseren Rollcontainers angeordnete Schwenktür aufweist.

Die CN 107583069 A beschreibt einen mobilen Desinfektionsschrank, welcher einen Schrankkörper, eine Desinfektionsvorrichtung und Bewegungsvorrichtungen umfasst. Die Desinfektionsvorrichtung befindet sich im Schrankkörper und ist fest mit dem Schrankkörper verbunden, und die Bewegungsvorrichtungen sind am Boden des Schrankkörpers angeordnet.

Die JP 2016123775 A beschreibt eine grundsätzlich ortsfeste Dampfsterilisationsvorrichtung, in welche ein als Rollwagen ausgebildeter Sterilisationsbehälter, der zur Aufnahme eines zu sterilisierenden Gegenstandes vorgesehen ist, ein- und ausgefahren werden kann.

### Darstellung der Erfindung

Aufgabe der vorliegenden Erfindung ist es, ein verbessertes System bereitzustellen, das insbesondere zur Versorgung einer Reinraumstation geeignet ist. Eine weitere Aufgabe der Erfindung ist es, ein Verfahren zur Versorgung einer Reinraumstation mit sterilen Gegenständen mittels des erfindungsgemässen

Rollcontainersystems anzugeben.

Gelöst werden diese Aufgaben durch das im Anspruch 1 definierte Rollcontainersystem sowie durch das im Anspruch 12 definierte Verfahren.

Das erfindungsgemässe Rollcontainersystem, welches insbesondere zur Versorgung einer Reinraumstation vorgesehen und geeignet ist, umfasst einen dichtend verschliessbaren äusseren Rollcontainer und mindestens einen darin ein- und ausfahrbaren inneren Rollcontainer. Der innere Rollcontainer weist ein im wesentlichen quaderförmiges Innengehäuse, welches auf einem ersten Lenkrollenpaar sowie einem in einer Laufrichtung des inneren Rollcontainers ausgerichteten ersten Zusatzrollenpaar läuft, sowie mindestens eine Ablagefläche, welche von einer parallel zur Laufrichtung des inneren Rollcontainers angeordneten Bedienungsseite her zugänglich ist, auf. Der äussere Rollcontainer weist ein im wesentlichen quaderförmiges Aussengehäuse, welches auf einem zweiten Lenkrollenpaar sowie einem in einer Laufrichtung des äusseren Rollcontainers ausgerichteten zweiten Zusatzrollenpaar läuft, sowie eine parallel zur Laufrichtung des äusseren Rollcontainers angeordnete Seitenwand, welche mit einer als Zugang zur Bedienungsseite eines im äusseren Rollcontainer befindlichen inneren Rollcontainers ausgebildeten seitlichen Schwenktür ausgestattet ist, und eine quer zur Laufrichtung des äusseren Rollcontainers angeordnete rückwärtige Schwenktür sowie ein innerhalb derselben angeordnetes ausklappbares Rampenelement für den inneren Rollcontainer, welches um eine in Bodennähe des äusseren Rollcontainers befindliche Schwenkachse schwenkbar angeordnet ist und mit mindestes einem Längsführungselement für den inneren Rollcontainer ausgestattet ist, auf. Weiterhin sind Transportsicherungsmittel vorhanden, um eine Relativbewegung des inneren Rollcontainers gegenüber dem äusseren Rollcontainer freigebbar zu blockieren.

Das erfindungsgemässe Verfahren zur Versorgung einer Reinraumstation mit sterilen Gegenständen mittels des erfindungsgemässen Rollcontainersystems umfasst die folgenden Schritte:
- ein unbeladener innerer Rollcontainer wird in einen äusseren Rollcontainer eingefahren;
- der Innenraum des äusseren Rollcontainers einschliesslich des darin befindlichen inneren Rollcontainers und des eingeklappten Rampenelementes wird desinfiziert und die rückwärtige Schwenktür verschlossen;
- der äussere Rollcontainer wird in eine Ladestation gebracht, wobei zumindest die mit einer seitlichen Schwenktür ausgestattete Seitenwand in einen durch partikelfiltrierte Luft laminar durchströmten Reinbereich der Ladestation positioniert wird,
- der Reinbereich einschliesslich des darin befindlichen Teils des äusseren Rollcontainers wird desinfiziert;
- die seitliche Schwenktür wird geöffnet und die im Reinbereich bereitgestellten sterilen Gegenstände auf die Ablagefläche des inneren Rollcontainers gelegt;
- die seitliche Schwenktür wird geschlossen;
- das Rollcontainersystem wird zur Reinraumstation gefahren und dort vor einem Eingang eines zugehörigen reinen Entladebereichs positioniert;
- die rückwärtige Schwenktür des äusseren Rollcontainers wird geöffnet und das Rampenelement ausgeklappt, wobei dessen Endbereich auf einen Bodenabschnitt des reinen Entladebereichs zu liegen kommt;
- der beladene innere Rollcontainer wird über das Rampenelement ohne Berührung unreiner Flächen in den Entladebereich gefahren;
- das Rampenelement wird eingeklappt, die rückwärtige Schwenktür verschlossen und der äussere Rollcontainer weggefahren.

Ein wesentlicher Aspekt der vorliegenden Erfindung liegt im ausklappbaren Rampenelement, mittels welchem eine Brücke vom Innenraum des äusseren Rollcontainers bis zu einem Bodenabschnitt des reinen Entladebereichs gebildet wird. Damit lässt sich der innere Rollcontainer ohne Berührung von unreinen Flächen über das in Reinzustand befindliche Rampenelement in den reinen Entladebereich fahren und dort abliefern.

Die Begriffe "oben", "unten", "seitlich" sind im Zusammenhang mit einer Vorrichtung in betriebsbereitem Zustand zu verstehen. Insbesondere wird "unten" stets für die mit Rollen versehene Seite eines Containers zu verstehen sein.

Im vorliegenden Zusammenhang sind geometrische Begriffe wie "parallel", "quaderförmig", "Schwenkachse" und dergleichen nicht im strengen mathematischen Sinn, sondern im Kontext der vorgesehenen Anwendung zu verstehen. Demnach kann beispielsweise ein Containergehäuse der hier beschriebenen Art auch noch dann als "quaderförmig" bezeichnet werden, wenn es von der geometrischen Idealform um einige Zentimeter abweicht.

Lenkrollen für Rollcontainer aller Art sind grundsätzlich bekannt. Die beim erfindungsgemässen System vorgesehenen Zusatzrollen können grundsätzlich als nichtdrehbare, sog. Bockrollen ausgestaltet sein, aber es kann sich auch um Lenkrollen handeln. In letzterem Fall ist das Merkmal "in Laufrichtung des Rollcontainers ausgerichtet" im Sinne von "in Laufrichtung des Rollcontainers ausrichtbar" zu verstehen.

Die erfindungsgemässe Ein- und Ausfahrbarkeit des inneren Rollcontainers in den äusseren Rollcontainer bedeutet, dass der Innenraum des äusseren Rollcontainers gross genug sein muss, um den zugeordneten inneren Rollcontainer vollständig aufzunehmen derart, dass bei eingeklapptem Rampenelement die rückwärtige Schwenktür und die seitliche Schwenktür verschlossen werden können. Es versteht sich, dass zur optimalen Nutzung der gegebenen Platzverhältnisse die Aussenabmessungen des inneren Rollcontainers in aller Regel möglichst gross gewählt werden. Weiterhin impliziert die Ein- und Ausfahrbarkeit, dass die relativen Abmessungen sämtlicher Rollen des inneren Rollcontainers in Bezug auf das Längsführungselement derart gewählt sind, dass die Ein- und Ausfahrbarkeit nicht behindert wird.

Die Wahl der geeigneten Materialien und Materialstärken bzw. Abmessungen für die einzelnen Bestandteile des Rollcontainersystems entspricht den im Fachgebiet bekannten Kriterien. Es versteht sich beispielsweise, dass die Wahl der Lenkrollen und Zusatzrollen durch die Grösse und das Gewicht des Rollcontainersystems beeinflusst wird.

Die dichtende Verschliessbarkeit des äusseren Rollcontainers bedeutet insbesondere, dass die Randzonen der vorgesehenen Schwenktüren mit einer geeigneten Dichtung versehen sind. Insbesondere soll dadurch der Eintritt von Mikroorganismen während der Fahrt durch eine unreine Zone verhindert werden.

Der erfindungsgemäss vorgesehene Reinbereich der Ladestation wird in an sich bekannter Weise durch partikelflitrierte Luft laminar durchströmt. Zu diesem Zweck ist die Luftzufuhrvorrichtung mit einem geeigneten Filtersystem ausgestattet. Bekanntlich kommen hierfür sogenannten HEPA ("High Efficiency Particulate Air") Filter zum Einsatz.

Die beim erfindungsgemässen Verfahren vorgesehenen Desinfektionsschritte werden auf grundsätzlich bekannte Art und Weise durchgeführt, wobei dies insbesondere ein Besprühen oder ein Abwischen der betreffenden Flächen und Bauteile mit entsprechenden Desinfektionsmitteln umfasst.
Vorteilhafte Ausgestaltungen und Ausführungsformen sind in den abhängigen Ansprüchen definiert.

Grundsätzlich ist das erfindungsgemässe Verfahren zur Versorgung von Reinraumstationen aller Art einsetzbar. Gemäss einer vorteilhaften Ausführungsform (Anspruch 13) ist die Reinraumstation mit einem Operationssaal verbunden.

Prinzipiell könnte die Längsführung des inneren Rollcontainers innerhalb des äusseren Rollcontainers auf verschiedene Arten bewerkstelligt werden. Vorteilhafterweise (Anspruch 2) ist das Längsführungselement mit mindestens einer Spurrille zur Aufnahme von je einer ersten Lenkrolle und ersten Zusatzrolle ausgebildet. Vorzugsweise sind zwei zueinander parallel verlaufende Spurrillen vorhanden, da sich hiermit eine zuverlässigere und stabilere Führung erzielen lässt. Besonders vorteilhaft ist es zudem (Anspruch 3), wenn jede Spurrille in einem von der Drehachse abgewandten Endbereich auseinanderläuft, um eine dort befindliche Lenkrolle drehmässig freizugeben. Damit lässt sich eine Längsführung realisieren, bei der die Drehbarkeit der jeweiligen Lenkrollen im Inneren des äusseren Rollcontainers verhindert ist und somit ein höchst unerwünschtes seitliches Ausscheren des inneren Rollcontainers, welches insbesondere beim Wechsel zwischen Vorwärts- und Rückwärtsfahrt auftritt, unterbunden wird. Durch die bevorzugte Ausgestaltung des Endbereichs der Spurrille wird einerseits die Drehbarkeit der Lenkrollen beim Ausfahren des inneren Rollcontainers frei gegeben. Andererseits wird beim Einfahren des inneren Rollcontainers eine Ausrichtung der Lenkrollen in die erwünschte Längsrichtung bewirkt.

Vorteilhaft ist es zudem (Anspruch 4), wenn der innere Rollcontainer an einer quer zur Bedienungsseite angeordneten Rückwand mit ersten Führungsgriffen versehen ist. Damit wird das Ein- bzw. Ausfahren des inneren Rollcontainers erleichtert. Besonders vorteilhaft ist es dabei (Anspruch 5), wenn die ersten Führungsgriffe als Griffbügel ausgebildet sind, die innerhalb des Umrisses des Innengehäuses verlaufen und mit zugehörigen Aussparungen des Innengehäuses ausgebildet sind. Dadurch lässt sich eine vorstehende Struktur vermeiden und entsprechend eine bessere Nutzung der maximal möglichen Aussenabmessungen des inneren Containers erzielen.

Vorteilhaft ist es ebenso (Anspruch 6), wenn der äussere Rollcontainer an der zur rückwärtigen Seitentür gegenüberliegenden Frontseite mit zweiten Führungsgriffen versehen ist. Damit wird grundsätzlich das präzise Bewegen des äusseren Rollcontainers erleichtert. Besonders vorteilhaft ist es dabei (Anspruch 7), wenn die zweiten Führungsgriffe als vertikale Griffelemente ausgebildet sind, die im Wesentlichen entlang einer zugeordneten Kante des Aussengehäuses verlaufen und mit zugehörigen Aussparungen des Aussengehäuses versehen sind. Damit wird ein unerwünschtes Einhängen von hervorstehenden Griffteilen vermieden.

Gemäss einer vorteilhaften Ausgestaltung (Anspruch 8) ist das Innengehäuse auf der Bedienungsseite durch mindestens ein Wandelement gebildet, das in ein Rahmenelement des inneren Rollcontainers ein- und aushängbar ist. Vorzugsweise ist für jede Ablagefläche des inneren Rollcontainers ein solches Wandelement vorgesehen, d.h. durch Aushängen eines Wandelementes wird eine zugehörige Ablagefläche zugänglich gemacht.

Vorteilhaft ist es überdies (Anspruch 10), wenn das Rampenelement mit einer Dämpfervorrichtung zur Dämpfung der Schwenkbewegung versehen ist. Es versteht sich, dass das Rampenelement entsprechend dem Gewicht und den Abmessungen des vorgesehenen inneren Rollcontainers genügend formstabil sein muss und dementsprechend ein nicht unerhebliches Gewicht haben wird. Durch eine entsprechende Dämpfervorrichtung wird ein unsanfter Aufprall beim Ausklappen des Rampenelementes vermieden. Vorteilhaft ist es zudem, wenn das Rampenelement mit einem Bedienungselement beispielsweise in Form eines Handgriffs versehen ist.

Gemäss einer weiteren vorteilhaften Ausgestaltung des Rollcontainersystems (Anspruch 10) umfassen die Transportsicherungsmittel:
- mindestens ein vorzugsweise gummielastisches Zapfenglied, das in Laufrichtung des inneren Rollcontainers in eine zugeordnete Aufnahme eingreift, wenn der innere Rollcontainer vollständig in den äusseren Rollcontainer eingefahren ist, und/oder
- eine durch zusammenwirkende Anschlagsflächen gebildete Seitenführung, welche eine seitliche Kippbewegung des inneren Rollcontainers verhindert.

Vorteilhaft ist es zudem (Anspruch 11), wenn die vorhandenen Lenkrollen mit einer Arretierbremse ausgestattet sind. Damit kann ein unerwünschtes Wegrollen des jeweiligen Rollcontainers vermieden werden. Insbesondere kann ein Wegrollen des in einer Warteposition befindlichen äusseren Rollcontainers, aber auch ein Wegrollen des inneren Rollcontainers, welcher namentlich auch als fahrbare Zwischenablage beim Empfänger der ausgelieferten Gegenstände eingesetzt werden kann, verhindert.

Je nach vorgesehener Implementation der Erfindung ist vorgesehen, dass die zur Bedienung des Systems vorgesehenen Schwenktüren mit Betätigungs- bzw. Freigabesicherungen ausgestattet sind, um eine Bedienung durch Unbefugte zu vermeiden.

Das Rollcontainersystem wird hier im Hinblick auf einen vollständig händischen Betrieb beschrieben. Dies schliesst jedoch einen angetriebenen bzw. motorisierten Betrieb nicht aus. Insbesondere kann für die Beförderung des äusseren Rollcontainers in gewissen Streckenabschnitten eine angetriebene Ausführungsform vorgesehen werden.

### Kurze Beschreibung der Zeichnungen

Ausführungsbeispiele der Erfindung werden nachfolgend anhand der Zeichnungen näher beschrieben, dabei zeigen:
- Fig. 1: ein innerer Rollcontainer, in perspektivischer Ansicht;
- Fig. 2: ein äusserer Rollcontainer, in perspektivischer Ansicht;
- Fig. 3: der innere Rollcontainer der Fig. 1, in einer seitlichen Ansicht zur Bedienungsseite;
- Fig. 4: der innere Rollcontainer der Fig. 1, in einer seitlichen Ansicht zur Rückseite;
- Fig. 5: der innere Rollcontainer der Fig. 1, in einer Schnittdarstellung gemäss dem Schnitt A-A der Fig. 3;
- Fig. 6: der innere Rollcontainer der Fig. 1, in einer Schnittdarstellung gemäss dem Schnitt B-B der Fig. 3;
- Fig. 7: der äussere Rollcontainer der Fig. 2, in einer seitlichen Ansicht zur Rückseite;
- Fig. 8: der äussere Rollcontainer der Fig. 2, in einer seitlichen Ansicht zu der als Zugang zu einem darin befindlichen inneren Rollcontainer vorgesehenen Seite;
- Fig. 9: der äussere Rollcontainer der Fig. 2, in einer Ansicht zur Unterseite;
- Fig. 10: der äussere Rollcontainer der Fig. 2, in einer seitlichen Ansicht zur Frontseite;
- Fig. 11: der äussere Rollcontainer der Fig. 2 mit einem darin befindlichen inneren Rollcontainer, in einer Schnittdarstellung gemäss dem Schnitt A-A der Fig. 8;
- Fig. 12: der äussere Rollcontainer der Fig. 2, in Draufsicht;
- Fig. 13: der äussere Rollcontainer der Fig. 2 mit einem darin befindlichen inneren Rollcontainer, in einer Schnittdarstellung gemäss dem Schnitt C-C der Fig. 8;
- Fig. 14: der äussere Rollcontainer der Fig. 2 mit einem darin befindlichen inneren Rollcontainer, in einer Schnittdarstellung gemäss dem Schnitt B-B der Fig. 12; und
- Fig. 15: ein Ausschnitt des Rollcontainersystems mit ausgeklapptem Rampenelement, in perspektivischer Ansicht.

### Wege zur Ausführung der Erfindung

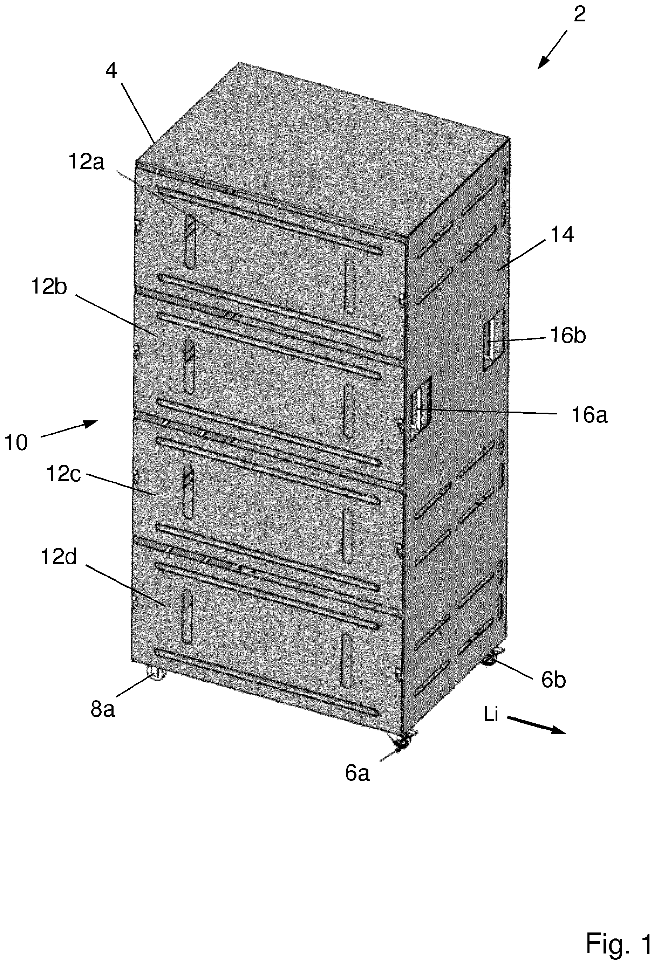

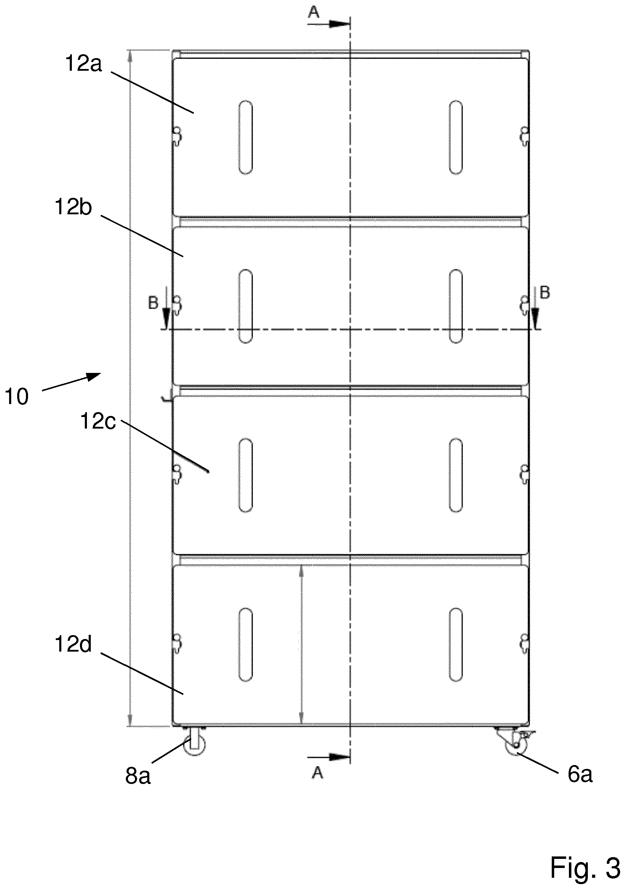

Der in den Fig. 1 sowie 3 bis 6 dargestellte innere Rollcontainer 2 weist ein im wesentlichen quaderförmiges Innengehäuse 4 auf, welches auf einem ersten Lenkrollenpaar 6a, 6b sowie einem in einer Laufrichtung des inneren Rollcontainers ausgerichteten ersten Zusatzrollenpaar 8a, 8b läuft. Der innere Rollcontainer hat eine parallel zur Laufrichtung Li angeordnete Bedienungsseite 10, welche im gezeigten Beispiel durch vier ein- und aushängbare Wandelemente 12a, 12b, 12c, 12d gebildet wird. An einer quer zur Bedienungsseite angeordneten Rückwand 14 ist der innere Rollcontainer mit ersten Führungsgriffen 16a, 16b versehen, die im gezeigten Beispiel als Griffbügel innerhalb des Umrisses des Innengehäuses mit zugehörigen Aussparungen des Innengehäuses ausgebildet sind.

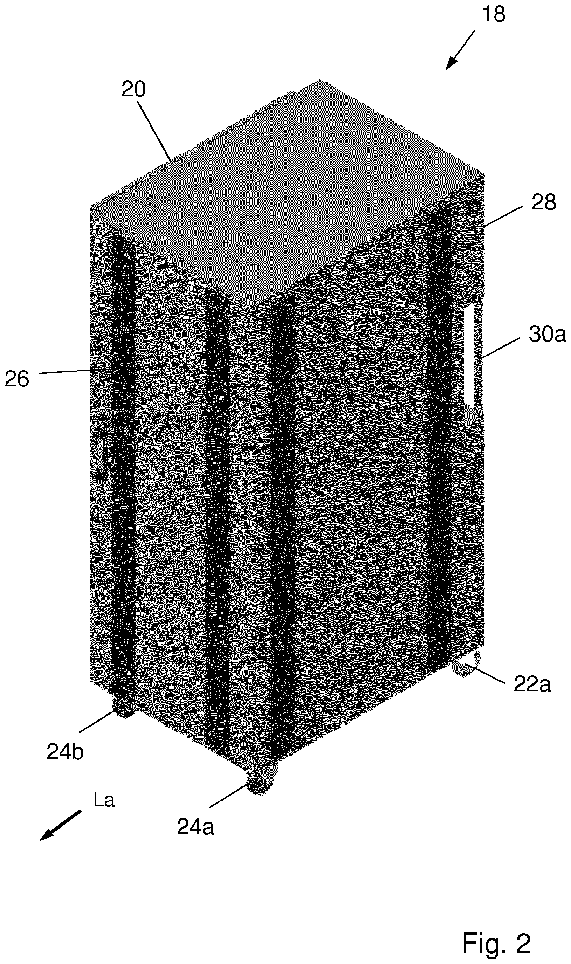

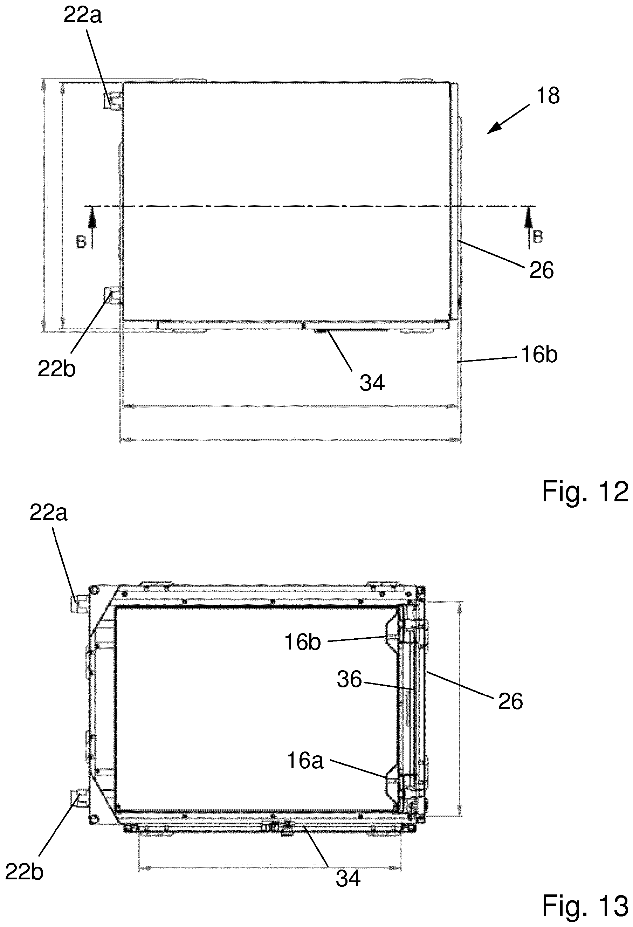

Der in den Fig. 2 sowie 7 bis 14 dargestellte äussere Rollcontainer 18 weist ein im wesentlichen quaderförmiges Aussengehäuse 20 auf, welches auf einem zweiten Lenkrollenpaar 22a, 22b sowie einem in einer Laufrichtung La des äusseren Rollcontainers ausgerichteten zweiten Zusatzrollenpaar 24a, 24b läuft. Ferner weist der äussere Rollcontainer eine quer zu seiner Laufrichtung La angeordnete rückwärtige Schwenktür 26 auf. An der zur rückwärtigen Schwenktür gegenüberliegenden Frontseite 28 ist der äussere Rollcontainer mit zweiten Führungsgriffen 30a, 30b versehen, die im gezeigten Beispiel als entlang einer zugeordneten Kante des Aussengehäuses verlaufende vertikale Griffelemente mit zugehörigen Aussparungen des Aussengehäuses ausgebildet sind.

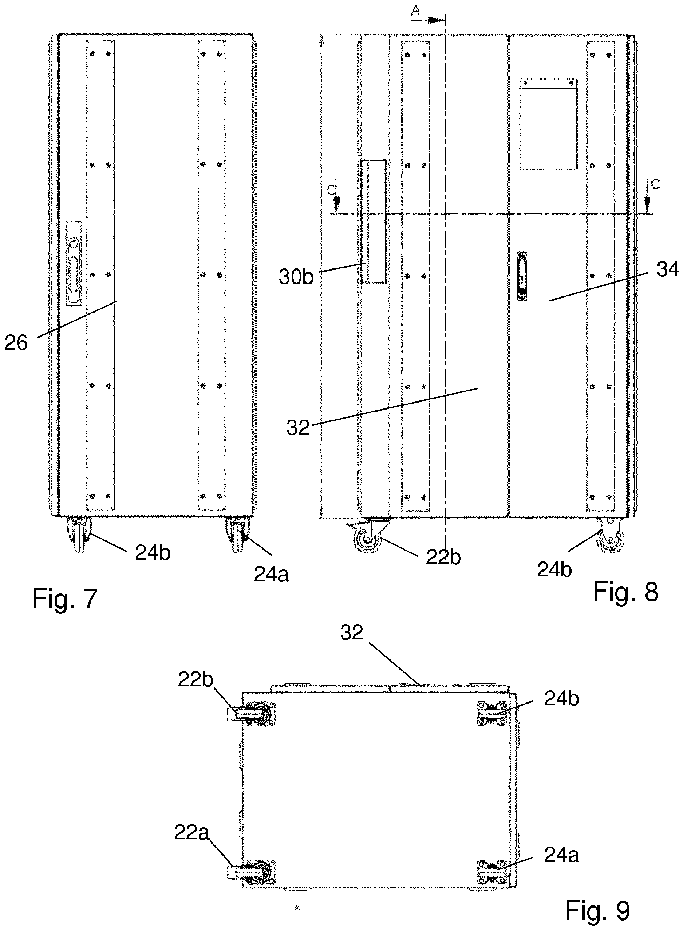

Wie aus der Fig. 8 ersichtlich ist, weist der zweite Container eine parallel zur Laufrichtung La angeordnete Seitenwand 32 auf, welche mit einer als Zugang zur Bedienungsseite eines im äusseren Rollcontainer befindlichen inneren Rollcontainers ausgebildeten seitlichen Schwenktür 34 ausgestattet ist.

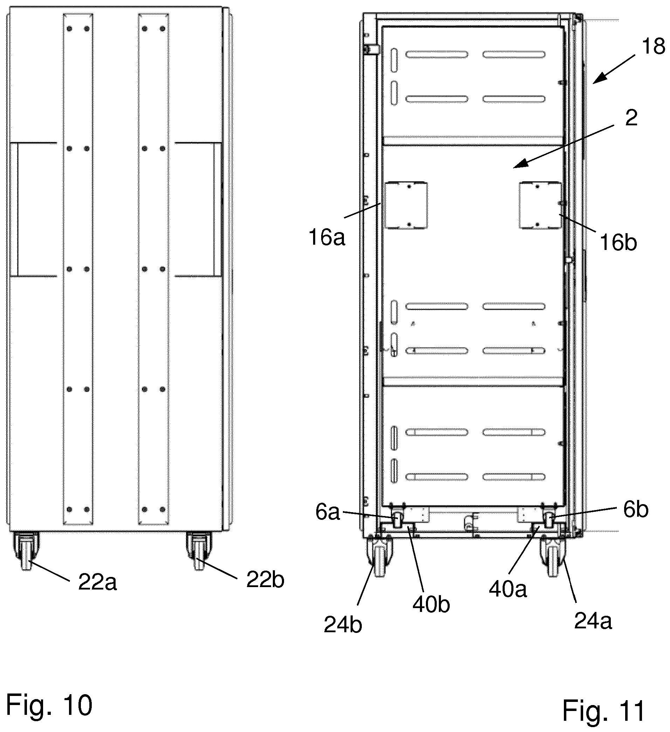

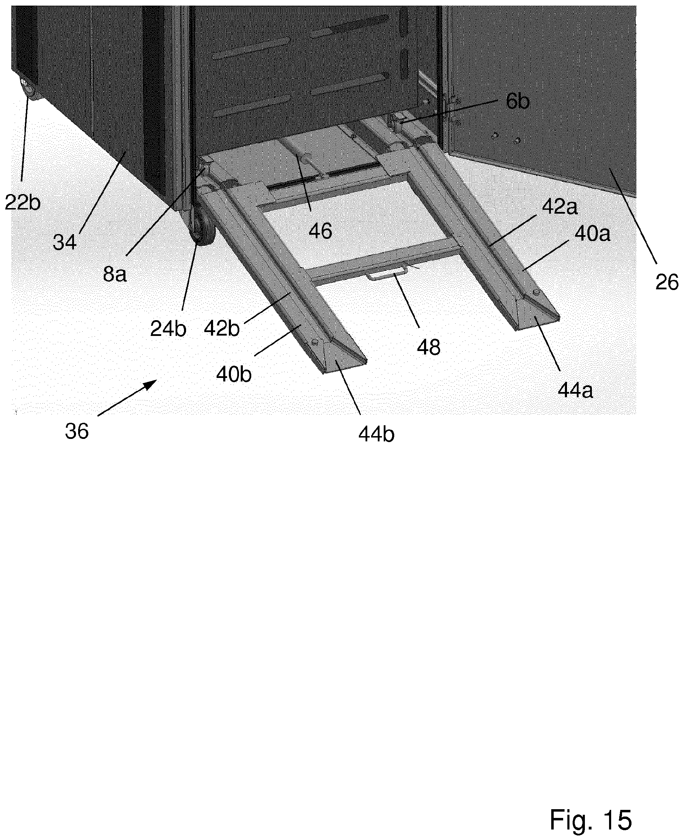

Das Zusammenwirken von innerem Rollcontainer 2 und äusserem Rollcontainer 18 ist aus den Fig. 11, 13, 14 und 15 ersichtlich. Der äussere Rollcontainer weist ein innerhalb der rückwärtigen Schwenktür 26 angeordnetes ausklappbares Rampenelement 36 für den inneren Rollcontainer auf, welches um eine in Bodennähe des äusseren Rollcontainers befindliche Schwenkachse 38 schwenkbar angeordnet ist. In gezeigten Beispiel ist das Rampenelement H-förmig ausgebildet und weist zwei laterale Längsführungselemente 40a, 40b für den inneren Rollcontainer auf. Jedes dieser Längsführungselemente weist einer Spurrille 42a, 42b auf, die zur Aufnahme von je einer ersten Lenkrolle und ersten Zusatzrolle des inneren Rollcontainers ausgebildet ist. Jede Spurrille ist in einem von der Drehachse abgewandten Endbereich 44a, 44b auseinanderlaufend ausgebildet, um eine dort befindliche Lenkrolle drehmässig freizugeben. Ausserdem ist das Rampenelement mit einer Dämpfervorrichtung 46 zur Dämpfung der Schwenkbewegung versehen und weist an einem Quersteg einen Handgriff 48 auf.

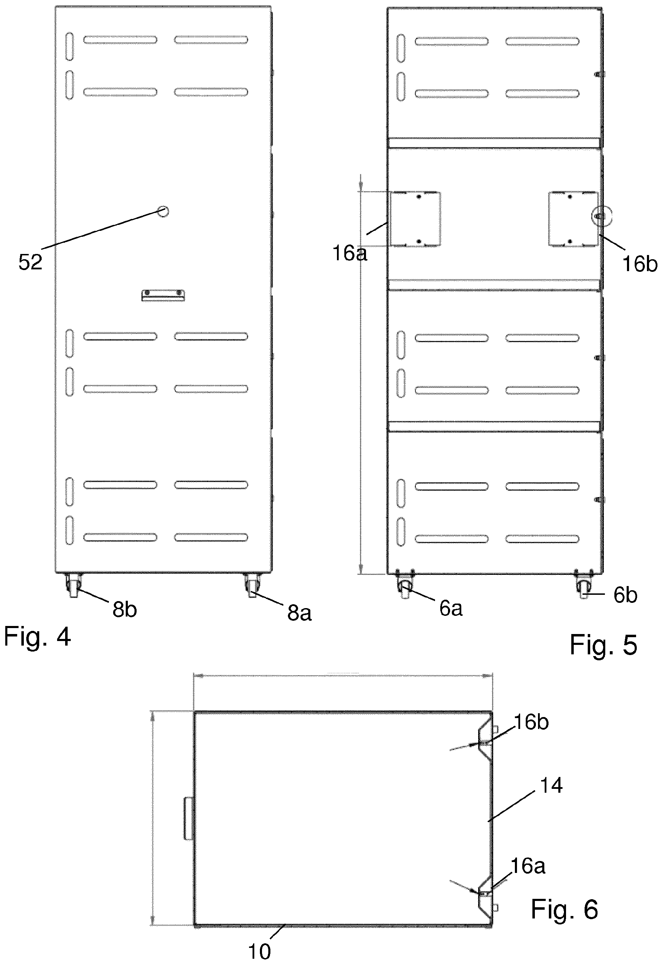

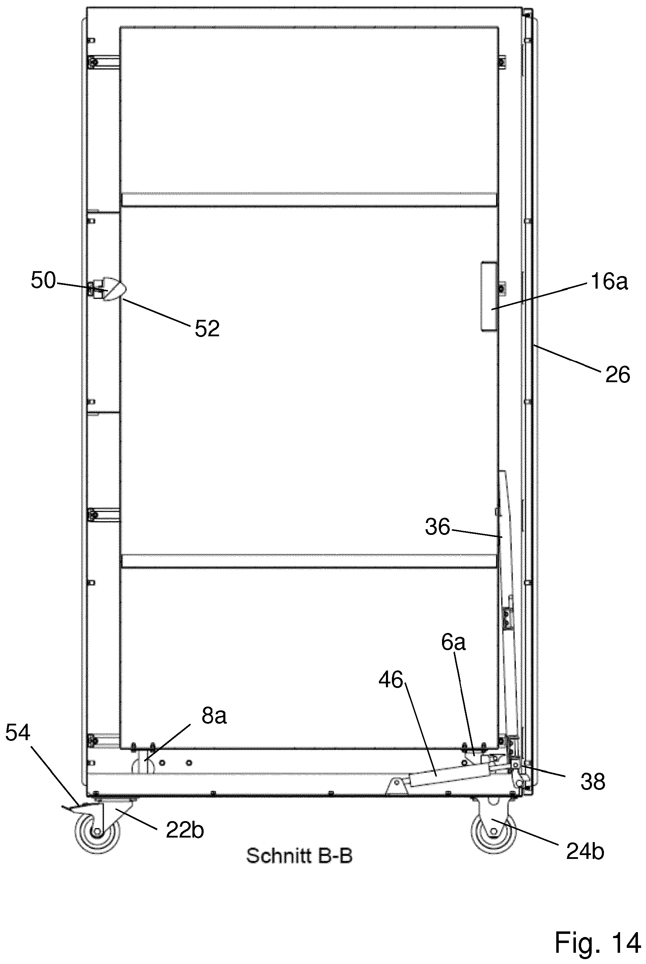

Wie aus den Fig. 4 und 14 ersichtlich, umfassen die Transportsicherungsmittel des Rollcontainersystems ein innerhalb des äusseren Rollcontainers angeordnetes Zapfenglied 50, das in Laufrichtung des inneren Rollcontainers vorstehend ausgebildet ist und in eine zugeordnete Aufnahme 52 des inneren Rollcontainers eingreift, wenn letzterer vollständig in den äusseren Rollcontainer eingefahren ist. Zur Vermeidung einer seitlichen Kippbewegung des inneren Rollcontainers ist überdies eine durch zusammenwirkende Anschlagsflächen gebildete Seitenführung vorgesehen, welche jedoch in den Figuren nicht näher dargestellt ist.

Zur Vermeidung eins unerwünschten Wegrollens der Rollcontainer sind die vorhandenen Lenkrollen vorteilhafterweise mit einer Arretierbremse 54 ausgestattet.

### Bezugszeichenliste

- 2: innerer Rollcontainer
- 4: Innengehäuse von 2
- 6a, 6b: erste Lenkrollen von 2
- 8a, 8b: erste Zusatzrollen von 2
- 10: Bedienungsseite von 2
- 12a, 12b,12c, 12d: Wandelemente von 2
- 14: Rückwand von 2
- 16a, 16b: erste Führungsgriffe von 2
- 18: äusserer Rollcontainer
- 20: Aussengehäuse von 18
- 22a, 22b: zweite Lenkrollen von 18
- 24a, 24b: zweite Zusatzrollen von 18
- 26: rückwärtige Schwenktür von 18
- 28: Frontseite von 18
- 30a, 30b: zweite Führungsgriffe von 18
- 32: Seitenwand von 18
- 34: seitliche Schwenktür von 18
- 36: Rampenelement von 18
- 38: Schwenkachse
- 40a, 40b: Längsführungselement
- 42a, 42b: Spurrille
- 44a, 44b: Endbereich
- 46: Dämpfervorrichtung
- 48: Handgriff
- 50: Zapfenglied
- 52: Aufnahme
- 54: Arretierbremse

## Patentansprüche

1. Rollcontainersystem, insbesondere zur Versorgung einer Reinraumstation, umfassend einen dichtend verschliessbaren äusseren Rollcontainer (18) und mindestens einen darin ein- und ausfahrbaren inneren Rollcontainer (2), wobei der innere Rollcontainer aufweist:
- ein im wesentlichen quaderförmiges Innengehäuse (4), sowie
- mindestens eine Ablagefläche, und
wobei der äussere Rollcontainer aufweist:
- ein im wesentlichen quaderförmiges Aussengehäuse (20), welches auf einem zweiten Lenkrollenpaar (22a, 22b) sowie einem in einer Laufrichtung (La) des äusseren Rollcontainers ausgerichteten zweiten Zusatzrollenpaar (24a, 24b) läuft, und
- eine quer zur Laufrichtung des äusseren Rollcontainers angeordnete Schwenktür (26),
**dadurch gekennzeichnet, dass**
- das Innengehäuse des inneren Rollcontainers auf einem ersten Lenkrollenpaar (6a, 6b) sowie einem in einer Laufrichtung (Li) des inneren Rollcontainers (2) ausgerichteten ersten Zusatzrollenpaar (8a, 8b) läuft,
- die Ablagefläche des inneren Rollcontainers von einer parallel zur Laufrichtung des inneren Rollcontainers angeordneten Bedienungsseite (10) her zugänglich ist,
- der äussere Rollcontainer eine parallel zur Laufrichtung des äusseren Rollcontainers angeordnete Seitenwand (32) aufweist, welche mit einer als Zugang zur Bedienungsseite eines im äusseren Rollcontainer befindlichen inneren Rollcontainers ausgebildeten seitlichen Schwenktür (34) ausgestattet ist,
- die quer zur Laufrichtung des äusseren Rollcontainers angeordnete Schwenktür (26) eine rückwärtige Schwenktür ist, und
- der äussere Rollcontainer ein innerhalb derselben angeordnetes ausklappbares Rampenelement (36) für den inneren Rollcontainer aufweist, welches um eine in Bodennähe des äusseren Rollcontainers befindliche Schwenkachse (38) schwenkbar angeordnet ist und mit mindestes einem Längsführungselement (40a, 40b) für den inneren Rollcontainer ausgestattet ist,
und dass:
- Transportsicherungsmittel vorhanden sind, um eine Relativbewegung des inneren Rollcontainers gegenüber dem äusseren Rollcontainer freigebbar zu blockieren.

2. Rollcontainersystem nach Anspruch 1, wobei das Längsführungselement (40a, 40b) mit mindestens einer Spurrille (42a, 42b) zur Aufnahme von je einer ersten Lenkrolle und ersten Zusatzrolle ausgebildet ist.

3. Rollcontainersystem nach Anspruch 2, wobei die Spurrille (42a, 42b) in einem von der Drehachse abgewandten Endbereich (44a, 44b) auseinanderläuft, um eine dort befindliche Lenkrolle drehmässig freizugeben.

4. Rollcontainersystem nach einem der vorangehenden Ansprüche, wobei der innere Rollcontainer an einer quer zur Bedienungsseite (10) angeordneten Rückwand (14) mit ersten Führungsgriffen (16a, 16b) versehen ist.

5. Rollcontainersystem nach Anspruch 4, wobei die ersten Führungsgriffe als Griffbügel innerhalb des Umrisses des Innengehäuses mit zugehörigen Aussparungen des Innengehäuses ausgebildet sind.

6. Rollcontainersystem nach einem der vorangehenden Ansprüche, wobei der äussere Rollcontainer an der zur rückwärtigen Schwenktür (26) gegenüberliegenden Frontseite (28) mit zweiten Führungsgriffen (30a, 30b) versehen ist.

7. Rollcontainersystem nach Anspruch 6, wobei die zweiten Führungsgriffe als vertikale Griffelemente ausgebildet sind, die im Wesentlichen entlang einer zugeordneten Kante des Aussengehäuses verlaufen und mit zugehörigen Aussparungen des Aussengehäuses versehen sind.

8. Rollcontainersystem nach einem der vorangehenden Ansprüche, wobei das Innengehäuse auf der Bedienungsseite (10) durch mindestens ein Wandelement (12a, 12b,12c, 12d) gebildet ist, das in ein Rahmenelement des inneren Rollcontainers ein- und aushängbar ist.

9. Rollcontainersystem nach einem der vorangehenden Ansprüche, wobei das Rampenelement (36) mit einer Dämpfervorrichtung (46) zur Dämpfung der Schwenkbewegung versehen ist.

10. Rollcontainersystem nach einem der vorangehenden Ansprüche, wobei die Transportsicherungsmittel umfassen:
- mindestens ein vorzugsweise gummielastisches Zapfenglied (50), das in Laufrichtung des inneren Rollcontainers in eine zugeordnete Aufnahme (52) eingreift, wenn der innere Rollcontainer vollständig in den äusseren Rollcontainer eingefahren ist, und/oder
- eine durch zusammenwirkende Anschlagsflächen gebildete Seitenführung, welche eine seitliche Kippbewegung des inneren Rollcontainers verhindert.

11. Rollcontainersystem nach einem der vorangehenden Ansprüche, wobei die besagten Lenkrollen mit einer Arretierbremse (54) ausgestattet sind.

12. Verfahren zur Versorgung einer Reinraumstation mit sterilen Gegenständen mittels des Rollcontainersystems nach einem der vorangehenden Ansprüche, umfassend die folgenden Schritte:
- ein unbeladener innerer Rollcontainer wird in einen äusseren Rollcontainer eingefahren;
- der Innenraum des äusseren Rollcontainers einschliesslich des darin befindlichen inneren Rollcontainers und des eingeklappten Rampenelementes wird desinfiziert und die rückwärtige Schwenktür verschlossen;
- der äussere Rollcontainer wird in eine Ladestation gebracht, wobei zumindest die mit einer seitlichen Schwenktür ausgestattete Seitenwand in einen durch partikelfiltrierte Luft laminar durchströmten Reinbereich der Ladestation positioniert wird,
- der Reinbereich einschliesslich des darin befindlichen Teils des äusseren Rollcontainers wird desinfiziert;
- die seitliche Schwenktür wird geöffnet und die im Reinbereich bereitgestellten sterilen Gegenstände auf die Ablagefläche des inneren Rollcontainers gelegt;
- die seitliche Schwenktür wird geschlossen;
- das Rollcontainersystem wird zur Reinraumstation gefahren und dort vor einem Eingang eines zugehörigen reinen Entladebereichs positioniert;
- die rückwärtige Schwenktür des äusseren Rollcontainers wird geöffnet und das Rampenelement ausgeklappt, wobei dessen Endbereich auf einen Bodenabschnitt des reinen Entladebereichs zu liegen kommt;
- der beladene innere Rollcontainer wird über das Rampenelement ohne Berührung unreiner Flächen in den Entladebereich gefahren;
- das Rampenelement wird eingeklappt, die rückwärtige Schwenktür verschlossen und der äussere Rollcontainer weggefahren.

13. Verfahren nach Anspruch 12, wobei die Reinraumstation mit einem Operationssaal verbunden ist.

## Claims

1. Roll container system, in particular for supplying a clean room station, comprising a sealingly closable outer roll container (18) and at least one inner roll container (2) which can be moved in and out thereof,
wherein the inner roll container comprises:
- a substantially cuboidal inner housing (4), and
- at least one storage surface, and
wherein the outer roll container comprises:
- a substantially cuboidal outer housing (20), which runs on a second pair of steering rollers (22a, 22b) and a second pair of additional rollers (24a, 24b) which are aligned in a running direction (La) of the outer roll container, and
- a pivot door (26) which is arranged transversely to the running direction of the outer roll container,
**characterized in that**
- the inner housing of the inner roll container runs on a first pair of steering rollers (6a, 6b) and a first pair of additional rollers (8a, 8b) which are aligned in a running direction (Li) of the inner roll container (2),
- the storage surface of the inner roll container is accessible from an operating side (10) which is arranged parallel to the running direction of the inner roll container,
- the outer roll container comprises a side wall (32) which is arranged parallel to the running direction of the outer roll container, which side wall is equipped with a lateral pivot door (34) designed as access to the operating side of an inner roll container located in the outer roll container,
- the pivot door (26) which is arranged transversely to the running direction of the outer roll container is a rear pivot door, and
- the outer roll container comprises a fold-out ramp element (36) for the inner roll container which is arranged therein, which ramp element is pivotably arranged around a pivot axis (38) located near the bottom of the outer roll container and is equipped with at least one longitudinal guide element (40a, 40b) for the inner roll container,
and that:
- transport securing means are provided for releasably blocking a relative movement of the inner roll container with respect to the outer roll container.

2. The roll container system according to claim 1, wherein the longitudinal guide element (40a, 40b) is configured with at least one track groove (42a, 42b) for receiving each of a first steering roller and a first additional roller.

3. The roll container system according to claim 2, wherein the track groove (42a, 42b) diverges in an end region (44a, 44b) facing away from the axis of rotation, in order to rotationally release a steering roller located therein.

4. The roll container system according to one of the preceding claims, wherein the inner roll container is provided, at a rear wall (14) thereof arranged transversely to the operator's side (10), with first guide handles (16a, 16b).

5. The roll container system according to claim 4, wherein the first guide handles are configured as handle bars within the outline of the inner housing with associated recesses in the inner housing.

6. The roll container system according to one of the preceding claims, wherein the outer roll container is provided, at the front side (28) thereof opposite to the rear pivot door (26), with second guide handles (30a, 30b).

7. The roll container system according to claim 6, wherein the second guide handles are configured as vertical handle elements, which are extending substantially along an associated edge of the outer housing and are provided with associated recesses of the outer housing.

8. The roll container system according to one of the preceding claims, wherein the inner housing on the operating side (10) is formed by at least one wall element (12a, 12b, 12c, 12d), which can be hooked into and unhooked from a frame element of the inner roller container.

9. The roll container system according to one of the preceding claims, wherein the ramp element (36) is provided with a damping device (46) for damping the pivoting movement.

10. The roll container system according to one of the preceding claims, wherein the transport securing means comprise:
- at least one, preferably rubber-elastic pin member (50), which engages in an associated receiving (52) in the running direction of the inner roll container, when the inner roll container is fully retracted into the outer roll container, and/or
- a lateral guide formed by cooperating stop surfaces, which prevents a lateral tilting movement of the inner roll container.

11. The roll container system according to one of the preceding claims, wherein the said steering rollers are provided with a locking brake (54).

12. A method for supplying sterile items to a clean room station by means of the roll container system according to one of the preceding claims, comprising the following steps:
- an unloaded inner roll container is moved into an outer roll container;
- the interior of the outer roll container including the inner roll container located therein and the folded-in ramp element are disinfected, and the rear pivot door is closed;
- the outer roll container is brought into a loading station, whereby at least the side wall equipped with a lateral pivot door is positioned in a clean region of the loading station through which particle-filtered air flows in a laminar manner,
- the clean region including the part of the outer roll container located therein is disinfected;
- the lateral pivot door is opened and the sterile items provided in the clean region are placed on the storage surface of the inner roll container;
- the lateral pivot door is closed;
- the roll container system is moved to the clean room station and positioned there in front of an entrance to an associated clean unloading region;
- the rear pivot door of the outer roll container is opened and the ramp element is folded out, whereby its end region comes to lay onto a floor section of the clean unloading region;
- the loaded inner roll container is moved over the ramp element into the unloading region without touching any unclean surfaces;
- the ramp element is folded in, the rear pivot door is closed and the outer roll container is driven away.

13. The method according to claim 12, wherein the clean room station is connected to an operating room.

## Revendications

1. Système de conteneur roulant notamment pour alimenter un poste de salle propre, comprenant un conteneur roulant extérieur (18) se fermant de manière étanche et au moins un conteneur roulant intérieur (2) rentrant et sortant,
- le conteneur roulant intérieur ayant :
* un caisson intérieur (4) essentiellement parallélépipédique ainsi qu'
* au moins une surface de dépose
- le conteneur roulant extérieur ayant :
* un caisson extérieur (20) essentiellement parallélépipédique circulant sur une seconde paire de roues directrices (22a, 22b) ainsi qu'une seconde paire de roues complémentaires (24a, 24b) alignées sur la direction de circulation (La) du conteneur roulant extérieur et,
* une porte pivotante (26) installée transversalement à la direction de circulation du conteneur roulant extérieur
système **caractérisé en ce que**
- le caisson intérieur du conteneur roulant intérieur circule sur une première paire de roues directrices (6a, 6b) ainsi qu'une première paire de roues complémentaires (8a, 8b) alignées sur la direction de circulation (Li) du conteneur roulant intérieur (2),
- la surface de dépose du conteneur roulant intérieur étant accessible par un côté de service (10) parallèle à la direction de circulation du conteneur roulant intérieur,
- le conteneur roulant extérieur ayant une paroi latérale (32) parallèle à la direction de circulation du conteneur roulant extérieur, et qui est équipée d'une porte latérale pivotante (34) pour l'accès au côté de service d'un conteneur roulant intérieur mis dans le conteneur roulant extérieur,
- la porte pivotante (26) installée transversalement à la direction de circulation du conteneur roulant extérieur étant une porte pivotante arrière, et
- le conteneur roulant extérieur ayant un élément de rampe (36) basculant à l'intérieur de celui-ci pour le conteneur roulant intérieur bascule autour d'un axe de basculement (38) à proximité du plancher du conteneur roulant extérieur et équipé d'au moins un élément de guidage longitudinal (40a, 40b) pour le conteneur roulant intérieur **en ce que**
- des moyens de sécurité de transport pouvant se libérer sont prévus pour bloquer le mouvement relatif du conteneur roulant intérieur par rapport au conteneur roulant extérieur.

2. Système de conteneur roulant selon la revendication 1,
dans lequel
l'élément de guidage longitudinal (40a, 40b) est réalisé avec au moins une rainure de circulation (42a, 42b) pour recevoir respectivement une première roue directrice et une première roue complémentaire.

3. Système de conteneur roulant selon la revendication 2,
dans lequel
la rainure de guidage (42a, 42b) s'élargit dans sa zone d'extrémité (44a, 44b) à l'opposé de l'axe de rotation, pour libérer en pivotement la roue directrice qui s'y trouve.

4. Système de conteneur roulant selon l'une des revendications précédentes,
dans lequel
le conteneur roulant intérieur est muni d'une paroi arrière (14) transversale au côté de service (10) et ayant des premières poignées de guidage (16a, 16b).

5. Système de conteneur roulant selon la revendication 4,
dans lequel
les premières poignées de guidage sont réalisées sous la forme d'arceaux de poignées s'inscrivant dans le contour du caisson intérieur avec des évidements correspondants du caisson intérieur.

6. Système de conteneur roulant selon l'une des revendications précédentes,
dans lequel
le conteneur roulant extérieur est muni de secondes poignées de guidage (30a, 30b) sur le côté frontal (28) à l'opposé de la porte pivotante arrière (26).

7. Système de conteneur roulant selon la revendication 6,
dans lequel
les secondes poignées de guidage sont réalisées sous la forme éléments de poignée verticaux qui sont pratiquement le long de l'arête associée du caisson extérieur et sont munis d'évidements correspondants du caisson extérieur.

8. Système de conteneur roulant selon l'une des revendications précédentes,
dans lequel
sur le côté de service (10) le caisson intérieur est formé par au moins un élément de paroi (12a, 12b, 12c, 12d) qui peut s'accrocher et se décrocher d'un élément de châssis du conteneur roulant intérieur.

9. Système de conteneur roulant selon l'une des revendications précédentes,
dans lequel
l'élément de rampe (36) est muni d'un dispositif amortisseur (46) pour amortir le mouvement de basculement.

10. Système de conteneur roulant selon l'une des revendications précédentes,
dans lequel
les moyens de sécurité de transport comprennent :
- au moins un organe en forme de goujon (50) de préférence élastique comme du caoutchouc et qui pénètre dans un logement associé (52) dans la direction de circulation du conteneur roulant intérieur lorsque le conteneur roulant intérieur est rentré complètement dans le conteneur roulant extérieur et/ou
- un guidage latéral formé par des surfaces de butée coopérant et qui évitent un mouvement de basculement latéral du conteneur roulant intérieur.

11. Système de conteneur roulant selon l'une des revendications précédentes,
dans lequel
les roues directrices sont équipées d'un frein de blocage (54).

12. Procédé d'alimentation d'un poste de salle propre avec des objets stérilisés à l'aide d'un système de conteneur roulant selon l'une des revendications précédentes, comprenant les étapes suivantes consistant à :
- rentrer un conteneur roulant intérieur non chargé, dans un conteneur roulant extérieur,
- fermer le volume intérieur du conteneur roulant extérieur y compris le conteneur roulant intérieur qui s'y trouve et désinfecter l'élément de rampe, relevé, et fermer la porte pivotante arrière,
- mettre le conteneur roulant extérieur dans un poste de chargement, en positionnant au moins la paroi latérale équipée d'une porte pivotante latérale dans une zone de nettoyage du poste de chargement, traversée par de l'air laminaire, filtré pour les particules,
- désinfecter la zone propre y compris la partie du conteneur roulant extérieur qui s'y trouve ;
- ouvrir la porte pivotante latérale et les objets stériles fournis à la zone propre sur la surface de dépose du conteneur roulant intérieur,
- fermer la porte pivotante latérale,
- conduire le système de conteneur roulant dans le poste de salle propre et le positionner devant l'entrée d'une zone de décharge propre correspondante,
- ouvrir la porte pivotante arrière du conteneur roulant extérieur et rabattre l'élément de rampe, dont la zone d'extrémité s'appuie sur un segment de sol de la zone de décharge propre ;
- conduire le conteneur roulant intérieur chargé, par l'élément de rampe, sans contact avec une surface non propre de la zone de décharge ;
- rabattre l'élément de rampe, fermer la porte pivotante arrière et évacuer le conteneur roulant extérieur.

13. Procédé selon la revendication 12,
selon lequel
on relie le poste de station propre à une salle d'opération.
